(19) Europäisches Patentamt
European Patent Office
Office européen des brevets

(11) EP 1 621 196 A1

(12) **EUROPEAN PATENT APPLICATION**
published in accordance with Art. 158(3) EPC

(43) Date of publication:
**01.02.2006 Bulletin 2006/05**

(21) Application number: **04721318.6**

(22) Date of filing: **17.03.2004**

(51) Int Cl.:
*A61K 31/443 (2000.01)* *A61P 17/00 (2000.01)*
*A61P 17/06 (2000.01)* *A61P 17/08 (2000.01)*
*C07D 405/06 (1990.01)*

(86) International application number:
**PCT/JP2004/003554**

(87) International publication number:
**WO 2004/082683 (30.09.2004 Gazette 2004/40)**

(84) Designated Contracting States:
**AT BE BG CH CY CZ DE DK EE ES FI FR GB GR HU IE IT LI LU MC NL PL PT RO SE SI SK TR**
Designated Extension States:
**AL LT LV MK**

(30) Priority: **17.03.2003 JP 2003071822**

(71) Applicant: **KYOWA HAKKO KOGYO CO., LTD.**
**Chiyoda-ku,**
**Tokyo 100-8185 (JP)**

(72) Inventors:
- **HARADA, Daisuke, c/o Pharm. Research Center Nagaizumo-cho,Sunto-gun,Shizuoka411-8731 (JP)**
- **MASAKI, Shigehiro, c/o Pharmac. Research Center Nagaizumo-cho,Sunto-gun,Shizuoka411-8731 (JP)**
- **MANABE, Haruhiko, c/o Pharmac. Resesarch Center, Nagaizumo-cho,Sunto-gun,Shizuoka411-8731 (JP)**

(74) Representative: **Casalonga, Axel**
**BUREAU D.A. CASALONGA - JOSSE**
**Paul-Heyse-Strasse 33**
**80336 München (DE)**

# (54) THERAPEUTIC AND/OR PREVENTIVE AGENT FOR CHRONIC SKIN DISEASE

(57) The present invention provides a therapeutic and/or preventive agent for chronic skin diseases which comprises 7-[2-(3,5-dichloro-4-pyridyl)-1-oxoethyl]-4-ethoxy-spiro[1,3-benzodioxol-2,1'-cyclopentane] or a pharmaceutically acceptable salt thereof as an active ingredient.

EP 1 621 196 A1

## Description

The present invention relates to therapeutic and/or preventive agents for chronic skin diseases (for example, contact dermatitis, atopic dermatitis, seborrheic dermatitis, nummular eczema, lichen simplex chronicus Vidal, autosensitization dermatitis, stasis dermatitis, asteatotic eczema, and psoriasis).

Phosphodiesterase (PDE) degrades cyclic adenosine 3',5'-monophosphate (cAMP) or cyclic guanosine 3',5'-monophosphate (cGMP) to regulate their concentrations in cells. PDE-IV, which is one of the PDE isozymes, is expressed in keratinocytes and inflammatory cells such as monocytes, macrophages, B-cells, T-cells and eosinophils (Br. J. Pharmacol., 1997, 121, p.221; J. Invest. Dermatol., 1985, 84, p.477; J. Pharmacol. Exp. Ther., 1994, 271, p.1167; J. Invest. Dermatol., 1998, 110, p287), and regulates cAMP or cGMP concentration. PDE-IV plays an important role for controlling inflammatory responses; i.e. regulation of the infiltration of inflammatory cells into inflammatory sites, the activation of the inflammatory cells, the activation of keratinocytes and the like (Mol. Pharmacol., 1995, 47, p.1164; Clin. Exp. Allergy, 1995, 25, p.616).

On the other hand, chronic skin diseases are considered to be induced or worsened by infiltration of inflammatory cells into skin lesions, activation of inflammatory cells in skin lesions, or activation of keratinocytes (J. Allergy Clin. Immunol., 2001, 107, p.871). Therefore, PDE-IV inhibitors are expected as therapeutic and/or preventive agents for chronic skin diseases.

For example, SB207499, which is one of the PDE-IV inhibitors, has been reported to inhibit a delayed-type allergic reaction in the skin in an animal model (Eur. J. Pharmacol., 2002, 446: 195). SB207499 is also reported to exhibit therapeutic effects on chronic dermatitis models (J. Pharmacol. Exp. Ther., 1998, 287, p.705).

Hitherto, 7-[2-(3,5-dichloro-4-pyridyl)-1-oxoethyl]-4-methoxy-spiro[1,3-benzodioxol-2,1'-cyclopentane] or a pharmaceutically acceptable salt thereof have been known to be used as PDE-IV inhibitors (WO 96/36624).

An object of the present invention is to provide a therapeutic and/or preventive agent for chronic skin diseases (for example, contact dermatitis, atopic dermatitis, seborrheic dermatitis, nummular eczema, lichen simplex chronicus Vidal, autosensitization dermatitis, stasis dermatitis, asteatotic eczema, and psoriasis) comprising 7-[2-(3,5-dichloro-4-pyridyl)-1-oxoethyl]-4-methoxy-spiro[1,3-benzodioxol-2,1'-cyclopentane] or a pharmaceutically acceptable salt thereof as an active ingredient.

The present invention relates to the following (1) to (6).

(1) A therapeutic and/or preventive agent for chronic skin diseases, which comprises 7-[2-(3,5-dichloro-4-pyridyl)-1-oxoethyl]-4-methoxy-spiro[1,3-benzodioxol-2,1'-cyclopentane] represented by Formula (I):

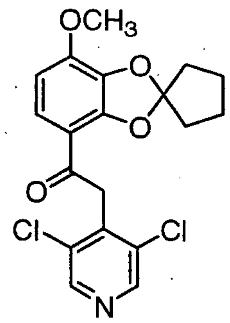

(I)

or a pharmaceutically acceptable salt thereof as an active ingredient.

(2) The therapeutic and/or preventive agent for chronic skin diseases according to (1), wherein the agent is an external preparation.

(3) A method for treating and/or preventing chronic skin diseases which comprises administering an effective amount of 7-[2-(3,5-dichloro-4-pyridyl)-1-oxoethyl]-4-methoxy-spiro[1,3-benzodioxol-2,1'-cyclopentane] represented by Formula (I):

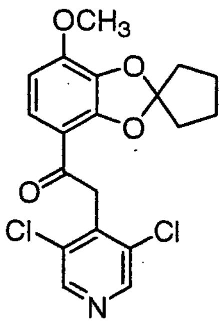

( I )

or a pharmaceutically acceptable salt thereof.

(4) The method for treating and/or preventing chronic skin diseases according to (3), wherein the method is characterized by administrating as an external preparation.

(5) Use of 7-[2-(3,5-dichloro-4-pyridyl)-1-oxoethyl]-4-methoxy-spiro[1,3-benzodioxol-2,1'-cyclopentane] represented by Formula (I):

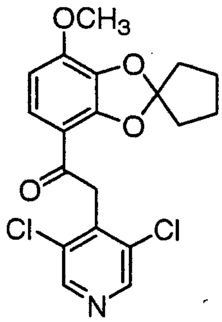

( I )

or a pharmaceutically acceptable salt thereof for manufacture of a therapeutic and/or preventive agent for chronic skin diseases.

(6) Use of 7-[2-(3,5-dichloro-4-pyridyl)-1-oxoethyl]-4-methoxy-spiro[1,3-benzodioxol-2,1'-cyclopentane] or the pharmaceutically acceptable salt thereof according to (5), wherein the therapeutic and/or preventive agent for chronic skin diseases is an external preparation.

The compound represented by Formula (I) is referred to as Compound (I) hereinafter.

Examples of the pharmaceutically acceptable salt of Compound (I) include pharmaceutically acceptable acid addition salts, metal salts, ammonium salts, organic amine addition salts, amino acid addition salts and the like.

Examples of the pharmaceutically acceptable acid addition salts of Compound (I) include inorganic acid salts such as a hydrochloride, a sulfate, a nitrate, a phosphate and the like; and organic acid salts such as an acetate, a maleate, a fumarate, a citrate and the like. Examples of the pharmaceutically acceptable metal salts include alkali metal salts such as a sodium salt, a potassium salt and the like; alkaline-earth metal salts such as a magnesium salt, a calcium salt and the like; an aluminum salt; a zinc salt and the like. Examples of the pharmaceutically acceptable ammonium salts include salts of ammonium, tetramethylammonium or the like. Examples of the pharmaceutically acceptable organic amine addition salts include addition salts of morpholine, piperidine or the like. Examples of the pharmaceutically acceptable amino acid addition salts include addition salts of glycine, phenylalanine, lysine, aspartic acid, glutamic acid or the like.

Next, a method for preparing Compound (I) will be described.

Compound (I) can be prepared by the method disclosed in WO 96/36624.

Among Compound (I), stereoisomers such as tautomers may be existed, and including such isomers, all possible isomers and mixtures thereof can be used as the therapeutic and/or preventive agents for chronic skin diseases of the present invention.

To obtain a salt of Compound (I), when Compound (I) is obtained in the form of a salt, it may be purified as it

is. When Compound (I) is obtained in the free form, Compound (I) may be dissolved or suspended in a suitable solvent, followed by addition of an acid or a base to form a salt. Then, the resulting salt may be isolated and purified.

Furthermore, Compound (I) and a pharmaceutically acceptable salt thereof may exist in the form of adducts with water or various solvents. These adducts can also be used as the therapeutic and/or preventive agents for chronic skin diseases of the present invention.

The pharmacological effects of Compound (I) will now be specifically described with the following Test Examples.

Test Example 1: The inhibitory effects on oxazolone-induced ear swelling in mice

Six-week-old BALB/c mice (male, Charles River Japan, Inc.) were used for the experiment. After at least one week quarantine and taming, seven-week-old mice which normally increased in weight and did not apparently show any abnormality were subjected to the experiment. The mice were placed in plastic cages (6 mice per cage) in a breeding room which was kept at a room temperature (19 to 25°C) and a humidity of 30 to 70% and was illuminated for 12 hours a day (from 7:00 a.m. to 7:00 p.m.). The mice were allowed to freely access commercially available pellets and water.

As an antigen solution, Oxazolone (Sigma-Aldrich) was dissolved in acetone (Kanto Kagaku) to prepare 0.5 weight/volume % of oxazolone-acetone solution. Compound (I) was dissolved in the antigen solution at 5 weight/volume % to prepare a Compound (I)-dissolving antigen solution. BALB/c mice were each applied with 100 μL of the antigen solution to the shaved abdomen for sensitization. Mice were shaved on abdomen on the previous day of the sensitization. The reaction was induced by an epicutaneous application of the antigen solution or the Compound (I)-dissolving antigen solution on the each side of the ear (10 μL on each site) 5 days after the sensitization. After the application, the applied sites were kept to dry by using a dryer. The mice applied with the antigen solution to induce the reaction were referred to as a solvent administration group, and the mice applied with the Compound (I)-dissolving antigen solution were referred to as a Compound (I) administration group. Mice that were not applied with any solution were referred to as a non-administration group. Ear thickness was measured with a dial thickness gauge (Ozaki Seisakusho) just before and 24 hours after the application for inducing the reaction, and the difference in the thickness was used as an indication of ear swelling. The inhibition ratio (%) against ear swelling was calculated using the following:

Inhibition ratio (%) = [{(value in solvent administration group) - (value in Compound (I) administration group)}/{(value in solvent administration group) - (value in non-administration group)}] ×100

The results are shown in Table 1.

Table 1

| Group | Dose (μg/site) | Auricular Edema (×0.01 mm) | Inhibition Ratio |
|---|---|---|---|
| Non-administration group | - | 1 | |
| Solvent administration group | - | 43## | |
| Compound (I) administration group | 100 | 16** | 64% |

##: P<0.01 (Wilcoxon rank-sum test, compared to the non-administration group)

**: P<0.01 (Wilcoxon rank-sum test, compared to the solvent administration group)

The ear swelling significantly increased in the solvent administration group compared with that in non-administration group (P=0.0049). On the other hand, in the Compound (I) administration group, significant inhibition of the increase of ear swelling was observed and the inhibition ratio was 64% (P=0.0051).

The above results show that the administration of Compound (I) can inhibit the ear swelling. Therefore, it is suggested that Compound (I) or a pharmaceutically acceptable salt thereof is useful as a therapeutic and/or preventive agent for chronic skin diseases.

Test Example 2: The inhibitory effects on the increase of ear thickness in a mouse model of dermatitis induced by

repeated application of oxazolone

Six-week-old BALB/c mice (male, Charles River Japan, Inc.) were used for the experiment. After at least one week quarantine and taming, seven-week-old mice which normally increased in weight and did not apparently show any abnormality were subjected to the experiment. The mice were placed in plastic cages (6 mice per cage) in a breeding room which was kept at a room temperature (19 to 25°C) and a humidity of 30 to 70% and was illuminated for 12 hours a day (from 7:00 a.m. to 7:00 p.m.). The mice were allowed to freely access commercially available pellets and water.
The experiment was performed according to a method by Kitagaki et al. (J. Invest. Dermatol., 1955, 105: 749) with small modification.
As an antigen solution, Oxazolone (Sigma-Aldrich) was dissolved in acetone (Kanto Kagaku) to prepare 0.5 weight/volume % of oxazolone-acetone solution. Compound (I) was dissolved in the antigen solution at 5 weight/volume % to prepare a Compound (I)-dissolving antigen solution. BALB/c mice were each applied with 10 μL of the antigen solution to the ear for sensitization. Then, 10 μL of the antigen solution or the Compound (I)-dissolving antigen solution was repeatedly applied to the same site of the ear at two or three days intervals through day 7 to 28 after the antigen sensitization. After the application, the applied sites were kept to dry by using a dryer. Mice which repeatedly applied with the antigen solution to induce the reaction were referred to as a solvent administration group, and mice repeatedly applied with the Compound (I)-dissolving antigen solution to induce the reaction were referred to as a Compound (I) administration group. Mice repeatedly applied with acetone were referred to as a non-administration group. On the 28th day when chronic dermatitis symptoms were observed, ear thickness was measured with a dial thickness gauge (Ozaki Seisakusho). The inhibition ratio (%) against the increase of ear thickness was calculated using the following:

Inhibition ratio (%) = [{(value in solvent administration group) - (value in Compound (I) administration group)}/{(value in solvent administration group) - (value in non-administration group)}] ×100

The results are shown in Table 2.

Table 2

| Group | Dose (μg/site/time) | Auricle thickness (×0.01 mm) | Inhibition Ratio |
|---|---|---|---|
| Non-administration group | - | 33 | |
| Solvent administration group | - | 91## | |
| Compound (I) administration group | 50 | 67** | 41% |

##: P<0.01 (Wilcoxon rank-sum test, compared to the non-administration group)

**: P<0.01 (Wilcoxon rank-sum test, compared to the solvent administration group)

The ear thickness significantly increased in the solvent administration group compared with the non-administration group (P=0.0075). In the Compound (I) administration group, the significant inhibition of the increase of ear thickness was observed and the inhibition ratio was 41% (P=0.0050).
These results show that the administration of Compound (I) can inhibit the increase of ear thickness with chronic dermatitis. Therefore, it is suggested that Compound (I) or a pharmaceutically acceptable salt thereof is useful as a therapeutic and/or preventive agent for chronic skin diseases.
Compound (I) or a pharmaceutically acceptable salt thereof may be administered by itself as it is, however, preferably it is usually provided as various pharmaceutical preparations. Also, these pharmaceutical preparations are used in animals and humans.
The pharmaceutical preparations according to the present invention may contain Compound (I) or the pharmaceutically acceptable salt thereof as an active ingredient solely or as a mixture with any other active ingredients for other treatment. Such pharmaceutical preparations are prepared by any process well known in the field of pharmaceutics by mixing the active ingredient with one or more pharmaceutically acceptable carriers.
As for an administration route, it is preferred to use the most effective administration route in treatment. Examples

of the administration routes include oral administration and parenteral administration such as percutaneous administration, intravenous administration and the like.

Examples of dosage form for administration include a tablet, an injection, an external preparation and the like.

Suitable dosage forms for oral administration, for example, tablet and the like, can be prepared by using excipients such as lactose, mannitol and the like, disintegrants such as starch and the like, lubricants such as magnesium stearate and the like, binders such as hydroxypropylcellulose and the like, surfactants such as fatty acid esters and the like, plasticizers such as glycerin and the like, antiseptics such as benzoic acid, p-hydroxybenzoic acid esters and the like, and the like.

Suitable Dosage forms for parenteral administration, for example, injections, are preferably prepared by using sterile aqueous preparations of active compounds which are isotonic with the blood of the recipients. For example, the solution for injection can be prepared by using a carrier of a salt solution, a glucose solution or a mixture of a salt solution and a glucose solution.

In above injections, diluents, flavors, and one or more additive(s) selected from the excipients, disintegrants, lubricants, binders, surfactants, plasticizers and antiseptics, which are exemplified in the oral administration may be added.

Suitable dosage forms for the external preparations, but not limited to, include preparations that are formed into cream, paste, jelly, gel, emulsion, liquid, or the like by dissolving or mixing and dispersing the active ingredient in base (e.g. ointments, liniments, lotions or the like); preparations that are formed by dissolving or mixing and dispersing the active ingredient and percutaneous absorption promoters in base, and then spreading them on supporting materials such as polyethylene, polyester, polyethylene terephthalate and the like(e.g. cataplasms, tapes or the like); and the like. Examples of above base include any pharmaceutically acceptable base, and known bases for ointments, liniments, lotions and the like can be used. Examples of such base include sodium alginate; polymers such as gelatin, corn starch, tragacanth gum, methylcellulose, hydroxyethyl cellulose, carboxymethyl cellulose, xanthan gum, dextrin, carboxymethyl starch, polyvinyl alcohol, sodium polyacrylate, methoxyethylene-maleic anhydride copolymer, polyvinyl ether, polyvinyl pyrrolidone and the like; fats and oils such as yellow beeswax, olive oil, cacao oil, sesame oil, soybean oil, camellia oil, peanut oil, beef tallow, lard, lanolin and the like; vaseline such as white vaseline, yellow vaseline and the like; paraffin; hydrocarbon gel ointments [for example, Plastibase[trade name(manufactured by Taisho Pharmaceutical Co., Ltd.)]; higher fatty acids such as stearic acid and the like; higher alcohols such as cetyl alcohol, stearyl alcohol and the like; polyethylene glycol; water and the like. Examples of above percutaneous absorption promoter include any pharmaceutically acceptable percutaneous absorption promoter, for example, alcohols such as methanol, ethanol, diethylene glycol, propylene glycol and the like; polar solvents such as dimethyl sulfoxide, dodecyl pyrrolidone and the like; urea; esters such as ethyl laurate, isopropyl myristate, cetyl octanoate and the like; azone; olive oil and the like. Additionally, inorganic fillers such as kaolin, bentonite, zinc oxide, titanium oxide and the like; viscosity- controlling agents; anti-aging agents; pH-controlling agents; humectants such as glycerin, propylene glycol and the like; and the like may be added to the base, if necessary.

Furthermore, the above external preparations may also contain diluents, flavors, and one or more additives selected from excipients, disintegrants, lubricants, binders, surfactants, plasticizers, antiseptics and the like, which are exemplified in the oral administration.

The dosage and the dosage frequency of Compound (I) or a pharmaceutically acceptable salt thereof may vary with the dosage forms, age and weight of patients, nature or severity of the symptom to be treated, and the like. In the oral administration, in general, a dose of 0.01 mg to 1 g, preferably, 0.05 to 50 mg, is administered to an adult patient once or several times a day. In the intravenous administration or the like, a dose of 0.001 to 100 mg, preferably, 0.01 to 10 mg, is administered to an adult patient once or several times a day. The external preparation (e.g. ointment, cream or the like) generally contains 1 to 1000 mg, preferably, 3 to 300 mg, of Compound (I) or a pharmaceutically acceptable salt thereof in 1 g of paste and is administered by applying it once or several times a day. However, these dosages and frequencies vary based on the above-mentioned various conditions.

The embodiments of the present invention will now be described with following Examples.

Example 1: Tablet

A tablet including the following composition is prepared by a conventional process. Compound (I) (40 g), lactose (286.8 g) and potato starch (60 g) are mixed, followed by adding 10% hydroxypropylcellulose aqueous solution (120 g) thereto. After the resulting mixture is kneaded, granulated, and dried according to a conventional process, the size of the granules is prepared for tablet pressing. The granules are mixed with magnesium stearate (1.2 g) and then pressed to make tablets (each tablet containing 20 mg of the active ingredient) by a tablet making machine having a striker of 8 mm diameter (Kikusui Co., Type RT-15).

| | | |
|---|---|---|
| Proscription | Compound (I) | 20 mg |
| | Lactose | 143.4 mg |
| | Potato starch | 30 mg |
| | Hydroxypropylcellulose | 6 mg |
| | Magnesium stearate | 0.6 mg |
| | | 200 mg |

Example 2: Injection

An injection including the following composition is prepared by a conventional process. Compound (I) (1 g) is dissolved in purified soybean oil, and purified egg-yolk lecithin (12 g) and glycerin (25 g) for injection are added thereto. Injectable distilled water is added to the resulting mixture to make the total volume 1000 mL, and the resulting mixture is kneaded and emulsified according to a conventional process. The resulting dispersion is filtered with a 0.2 μm disposable membrane filter under sterile condition and is dispensed into glass vials at a volume of 2 mL per vial (each vial contains 2 mg of the active ingredient) under the sterile condition to obtain the injections.

| | | |
|---|---|---|
| Prescription | Compound (I) | 2 mg |
| | Purified soybean oil | 200 mg |
| | Purified egg-yolk lecithin | 24 mg |
| | Glycerin for injection | 50 mg |
| | Injectable distilled water | 1.72 mL |
| | | 2.00 mL |

Example 3: External preparation (ointment)

A external preparation (ointment) including the following composition is prepared according to a conventional process. White Vaseline (65 g) is heated with stirring, and propylene glycol (25 g) is added thereto. To the resulting mixture, a mixture of Compound (I) (5 g) and cetyl octanoate (5 g) is added and dispersed with continuous stirring and heating. Then, the dispersion is gradually cooled to about 25°C and put into an appropriate container to obtain the external preparation (ointment).

| | | |
|---|---|---|
| Prescription | Compound (I) | 5 g |
| | White Vaseline | 65 g |
| | Propylene glycol | 25 g |
| | Cetyl octanoate | 5 g |
| | | 100 g |

The present invention provides a therapeutic and/or preventive agent for chronic skin diseases comprising 7-[2-(3,5-dichloro-4-pyridyl)-1-oxoethyl]-4-methoxy-spiro[1,3-benzodioxol-2,1'-cyclopentane] or a pharmaceutically acceptable salt thereof as an active ingredient.

## Claims

**1.** A therapeutic and/or preventive agent for chronic skin diseases, which comprises 7-[2-(3,5-dichloro-4-pyridyl)-1-oxoethyl]-4-methoxy-spiro[1,3-benzodioxol-2,1'-cyclopentane] represented by Formula (I):

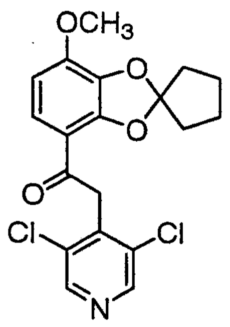

(I)

or a pharmaceutically acceptable salt thereof as an active ingredient.

2. The therapeutic and/or preventive agent for chronic skin diseases according to claim 1, wherein the agent is an external preparation.

3. A method for treating and/or preventing chronic skin diseases which comprises administering an effective amount of 7-[2-(3,5-dichloro-4-pyridyl)-1-oxoethyl]-4-methoxy-spiro[1,3-benzodioxol-2,1'-cyclopentane] represented by Formula (I):

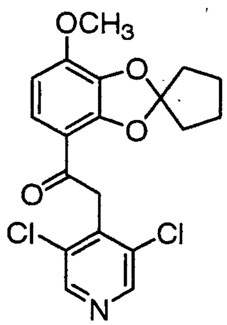

(I)

or a pharmaceutically acceptable salt thereof.

4. The method for treating and/or preventing chronic skin diseases according to claim 3, wherein the method is **characterized by** administrating as an external preparation.

5. Use of 7-[2-(3,5-dichloro-4-pyridyl)-1-oxoethyl]-4-methoxy-spiro[1,3-benzodioxol-2,1'-cyclopentane] represented by Formula (I):

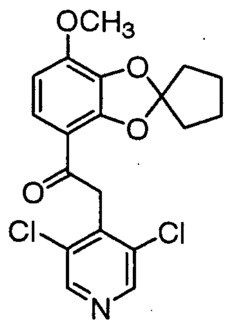

(I)

or a pharmaceutically acceptable salt thereof for manufacture of a therapeutic and/or preventive agent for chronic skin diseases.

**6.** Use of 7-[2-(3,5-dichloro-4-pyridyl)-1-oxoethyl]-4-methoxy-spiro[1,3-benzodioxol-2,1'-cyclopentane] or the pharmaceutically acceptable salt thereof according to claim 5, wherein the therapeutic and/or preventive agent for chronic skin diseases is an external preparation.

## INTERNATIONAL SEARCH REPORT

International application No.
PCT/JP2004/003554

**A. CLASSIFICATION OF SUBJECT MATTER**
Int.Cl[7] A61K31/443, A61P17/00, 17/06, 17/08 // C07D405/06

According to International Patent Classification (IPC) or to both national classification and IPC

**B. FIELDS SEARCHED**

Minimum documentation searched (classification system followed by classification symbols)
Int.Cl[7] A61K31/00-31/80, A61P17/00-17/08, C07D405/00-405/12

Documentation searched other than minimum documentation to the extent that such documents are included in the fields searched

Electronic data base consulted during the international search (name of data base and, where practicable, search terms used)
MEDLINE(STN), EMBASE(STN), BIOSIS(STN), BIOTECHABS(STN), CAplus(STN), REGISTRY(STN), WPI(DIALOG), JSTPLUS(JOIS), JMEDPLUS(JOIS)

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 96/36624 A1 (Kyowa Hakko Kogyo Co., Ltd.), 21 November, 1996 (21.11.96), Example 140; page 1, lines 4 to 9<br>& EP 771794 A1 & AU 9657029 A<br>& NO 9700151 A & KR 97704724 A<br>& US 2002/0128290 A1 & US 6514996 B2<br>& US 6716987 B1 & CN 1154697 A | 1,5<br>2,6 |
| Y | WO 99/64423 A1 (DARWIN DISCOVERY LTD.), 16 December, 1999 (16.12.99), Claims<br>& JP 2003-526601 A & AU 9942776 A<br>& US 6169090 B1 & EP 1086106 A1 | 1,2,5,6 |

[X] Further documents are listed in the continuation of Box C.  [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 April, 2004 (19.04.04) | 11 May, 2004 (11.05.04) |
| Name and mailing address of the ISA/<br>Japanese Patent Office<br><br>Facsimile No. | Authorized officer<br><br>Telephone No. |

Form PCT/ISA/210 (second sheet) (January 2004)

**INTERNATIONAL SEARCH REPORT**

International application No.
PCT/JP2004/003554

C (Continuation). DOCUMENTS CONSIDERED TO BE RELEVANT

| Category* | Citation of document, with indication, where appropriate, of the relevant passages | Relevant to claim No. |
|---|---|---|
| Y | WO 01/70738 A2 (MERCK FROSST CANADA & CO.), 27 September, 2001 (27.09.01), Claims & JP 2003-528099 A & AU 200142172 A & US 6399636 B2 & US 2002/0013347 A1 & US 2002/0156105 A1 & US 2002/0173661 A1 & EP 1272488 A2 | 1,2,5,6 |
| Y | WO 03/002511 A1 (Nikken Chemicals Co., Ltd.), 09 January, 2003 (09.01.03), Claims & JP 2003-81296 A & EP 1405844 A1 | 1,2,5,6 |
| Y | WO 03/016279 A1 (Tanabe Seiyaku Co., Ltd.), 27 February, 2003 (27.02.03), Claims & JP 2003-119196 A & JP 2003-116980 A & JP 2003-119195 A | 1,2,5,6 |
| Y | COHAN, V.L. et al., In Vitro Pharmacology of the Novel Phosphodiesterase Type 4 Inhibitor, CP-80633., J. Pharmacol.Exp.Ther., 1996, 278(3), pages 1356 to 1361; particularly, gist, Discussion | 1,2,5,6 |
| Y | GRISWOLD, D.E. et al., SB 207499 (Ariflo), a Second Generation Phosphodiesterase 4 Inhibitor, Reduces Tumor Necrosis Factor α and Interleukin-4 Production in vivo., J.Pharmacol.Exp.Ther., 1998, 287(2), pages 705 to 711; particularly, gist, Results, Discussion | 1,2,5,6 |
| Y | EHINGER, A.M. et al., Effects of the phosphodiesterase 4 inhibitor RPR 73401 in a model of immunological inflammation., Eur.J.Pharmacol., 2000, 392, pages 93 to 99; full text | 1,2,5,6 |
| Y | BAEUMER, W. et al., Effects of the phosphodiesterase 4 inhibitors SB 207499 and AWD 12-281 on the inflammatory reaction in a model of allergic dermatitis., Eur.J. Pharmacol., 2002, 446, pages 195 to 200; full text | 1,2,5,6 |
| Y | Arihiko KANEHIRO, "Allergy Shikkan - Chiryoyaku no Kongo no Tenbo: PDE4 Sogaiyaku", Mod.Phys., 2002, 22(4), pages 509 to 512; particualrly, abstract; page 511, left column, 8th line from the bottom to right column, line 14 | 1,2,5,6 |

Form PCT/ISA/210 (continuation of second sheet) (January 2004)

**INTERNATIONAL SEARCH REPORT**

International application No.
PCT/JP2004/003554

**Box No. II Observations where certain claims were found unsearchable (Continuation of item 2 of first sheet)**

This international search report has not been established in respect of certain claims under Article 17(2)(a) for the following reasons:

1. [X] Claims Nos.: 3, 4
because they relate to subject matter not required to be searched by this Authority, namely:
The inventions as set forth in Claims 3, 4 pertains to methods for treatment of the human body by or therapy.
(Article 17(2)(a)(i) of the PCT and Rule 39.1(iv) of the Regulations under the PCT)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest.

[ ] No protest accompanied the payment of additional search fees.

Form PCT/ISA/210 (continuation of first sheet (2)) (January 2004)